Europäisches Patentamt

European Patent Office

Office européen des brevets

(11) Publication number: **0 066 899**

**B1**

(12) **EUROPEAN PATENT SPECIFICATION**

(45) Date of publication of patent specification: 30.03.88

(51) Int. Cl.⁴: **A 61 F 13/02,** A 61 B 19/08

(21) Application number: **82105128.1**

(22) Date of filing: **11.06.82**

(54) Delivery system for adhesively affixed copolymer medical coverings.

(30) Priority: **10.06.81 US 272149**

(43) Date of publication of application:
. **15.12.82 Bulletin 82/50**

(45) Publication of the grant of the patent:
**30.03.88 Bulletin 88/13**

(84) Designated Contracting States:
**BE CH DE FR GB IT LI NL SE**

(56) References cited:
**EP-A-0 051 935**
**US-A-3 916 887**
**US-A-4 265 234**

(73) Proprietor: **Muchin, Jerome D.**
**16547 Park Lane Circle**
**Los Angeles California 90049 (US)**

(72) Inventor: **Muchin, Jerome D.**
**16547 Park Lane Circle**
**Los Angeles California 90049 (US)**

(74) Representative: **Vogeser, Werner, Dipl.-Ing.**
**et al**
**Patentanwälte Hansmann & Vogeser Albert-**
**Rosshaupter-Strasse 65**
**D-8000 München 70 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

Background of the Invention

The present invention relates to adhesively affixed copolymer medical coverings and in particular to a delivery system for enabling an adhesively attachable copolymer medical covering to be affixed to a subject without clinging, adhering or sticking to itself and which enables substantially the entire surface of the copolymer medical covering to be positioned in adhesive contact with the subject.

The use of polyurethane or other thin copolymer films in medical applications such as for surgical drapes, wound dressings, bangages, and the like, are known. For example, one polyurethane film is presently marketed under the trademark "Op-Site" by Smith & Nephews. This product is disclosed in US—A—3,645,835. The covering disclosed in the this patent includes an adhesively covered film sheet made of polyurethane or other copolymer. The adhesive is applied to one surface of the polyurethane film and is thereafter covered by a suitable protector material. The protector material or layer remains in place until just prior to affixation to the subject.

The film is adhesively affixed to the subject by first peeling a small portion of the protector material from the adhesive and then affixing the exposed adhesive to the body of the subject. Thereafter the adhesive and polyurethane is progressively and continuously pressed against the body of the biological subject as the backing paper is progressively removed.

This prior art delivery system thus left the back side of the polyurethane film sheet exposed and susceptible to germs, dirt and the like coming in contact with the back of the polyurethane film sheet. Additionally, because the polyurethane film sheet with adhesive is relatively thin and very flexible, it is impractical if not impossible to remove the protector entirely and thereafter place the polyurethane sheet on the subject. Such an application technique results in the gathering, clinging and self-sticking of portions of the polyurethane film sheet preventing the sheet from being affixed smoothly over a part of the body of the biological subject. To avoid this problem, it is necessary to effect affixation and removal of the protector from the adhesive at substantially the same time as above described.

Even utilizing the above-described application technique, however, it is difficult to affix polyurethane sheets having a thickness of aproximately 0,0127 mm. However, limitations as to the thinness of the polyurethane exist because of the inability to easily and conveniently apply the adhesively covered polyurethane film to the body of the subject without the above-described gathering and self-sticking. The problem is compounded as the surface area of the film sheet increased as is required for surgical drape applications even if the film sheet thickness increased to some $10^{-2}$ mm.

Additionally, it is desired to have as thin a sheet of polyurethane as possible for patient comfort. Specifically, such a thin polyurethane film stretches and moves with the body of the subject and allows the skin of the subject to stretch, contract and fold in a substantially unimpeded manner while still maintaining the covering over the desired part of the subject's body.

Another delivery system is disclosed in US—A—4,265,234 but the film sheet of this prior art is not coextensive with its backing. The backing and the release sheet are greater in both directions than the film sheet to provide package for it. The backing is adhesively affixed to the back surface of the film sheet. Finally, the backing does not support the entire back surface of the film sheet. It is only supporting a portion of the film because of the tab between film sheet and backing which prevents the backing from adhering to a portion of the film sheet or bandage.

Also the EP—A—0 051 935 discloses a delivery system, but the film sheet and the film sheet carrier are not coextensive, because the film sheet carrier, the releasable layer 7, extends beyond the film sheet to provide a grasping tab. The only method disclosed for achieving this structure is extrusion. The EP—A—0 051 935 does not explain how a film sheet could be cast onto the film sheet carrier in only certain regions. If the film sheet and the film sheet carrier can be coextensive it is easy to cast the film sheet onto the carrier and to dyecut (size) the both parts together. Furthermore, as film sheet and film sheet carrier are not coextensive in the EP 0 051 935 it is necessary that the rigid carrier is attached to the film sheet more tenaciously than the release sheet is attached to the adhesive surface of the film. This is a consequence of the way the carrier and the release sheet are grasped and pulled apart to effect the removal of the release sheet to expose the adhesive. Applicant's invention does not require such a relative adherence characteristic because the film sheet and the film sheet carrier are coextensive and both are grasped together in one hand while the other hand grasps and pulls free the release sheet.

The present invention solves the aforementioned problems by providing a delivery system with the characterizing features of claim 1. Advantageous embodiments of the invention are disclosed in the subclaims.

This delivery system comprises a film sheet of polyurethane or other copolymer material having a thickness up to some $10^{-2}$ mm or more which is stretchable and which has adhesive applied to one side and may be applied to the body of a biological subject without gathering or self-adhering. This result is accomplished by providing a relatively unstretchable and rigid film sheet carrier on the surface of the film sheet opposite the adhesively covered surface where the film sheet carrier remains in place until after the film sheet has been affixed to the biological subject. Suitable means, such as a carrier removal tab, is then provided so that the film sheet carrier can be easily removed and discarded leaving only the film sheet affixed by the adhesive to the body of the biological subject.

The above-described film sheet not only provides a substantially nonstretchable and relatively rigid carrier for the film sheet but also protects the film sheet from exposure to dirt, germs and the like.

Summary of the Invention

The present invention comprises a delivery system for adhesively attached copolymer medical coverings for covering parts of the body of a biological subject. The delivery system in accordance with the invention includes a film sheet for covering a selected part of the biological subject where the film sheet has a subject facing surface and an opposite parallel back surface. The film sheet back surface is releasably joined to a film sheet carrier. The film sheet carrier is inelastic and rigid relative to the film sheet but is bendable to conform generally to the contours of the part of the biological subject. The film sheet carrier is for the purpose of supporting and maintaining the film sheet in planar configuration until after the film sheet is affixed to cover the selected part of the biological subject. The film sheet carrier is thereafter removed and discarded. The delivery system further includes a release sheet and a transfer adhesive disposed for covering at least a portion of the release sheet. The release sheet and transfer adhesive are then positioned adjacent the film sheet so that the transfer adhesive adheres to at least a portion of the subject facing surface of the film sheet. Thus, the film sheet is sandwiched between the release sheet and the film sheet carrier prior to application to the selected part of the biological subject. In operation, the transfer adhesive transfers to the film upon removal of the release sheet prior to application of the film sheet to the selected part of the biological subject. The film sheet is held to the selected part of the subject by the transfer adhesive.

In accordance with the invention, the delivery system further includes a pull strip which is adhesively attached along at least a section of one edge of the subject facing surface of the film sheet. Preferably, at least a section of the release sheet is transfer adhesive-free with the transfer adhesive-free section being positioned over the pull strip whereby at least a portion of the pull strip is free of transfer adhesive to facilitate grasping and removal of the film sheet from the biological subject after use.

In accordance with the further embodiment of the invention, the delivery system includes a carrier removal tab which is adhesively attached to the film sheet carrier for being grasped and pulled to thereby pull the film sheet carrier from the back surface of the film sheet after placement of the film sheet on the selected part of the biological subject. The carrier removal tab is positioned so that the film sheet carrier is between the film sheet and the carrier removal tab. In the preferred embodiment, the carrier removal tab is relatively rigid compared to the film sheet carrier to facilitate grasping and pulling.

Brief Description of the Drawings

A complete understanding of the present invention and of the above and other advantages thereof may be gained from a consideration of the following description of the preferred embodiment taken in conjunction with the accompanying drawings in which:

FIGURE 1 is a cross-sectional view of the delivery system in accordance with the invention with layer thicknesses exaggerated.

FIGURES 2A, 2B and 2C illustrate one method of making the delivery system shown in FIGURE 1.

FIGURES 3A, 3B and 3C illustrate one method by which the delivery system in accordance with the invention may be used to adhesively affix a copolymer film sheet medical covering to a body part of a biological subject.

Detailed Description

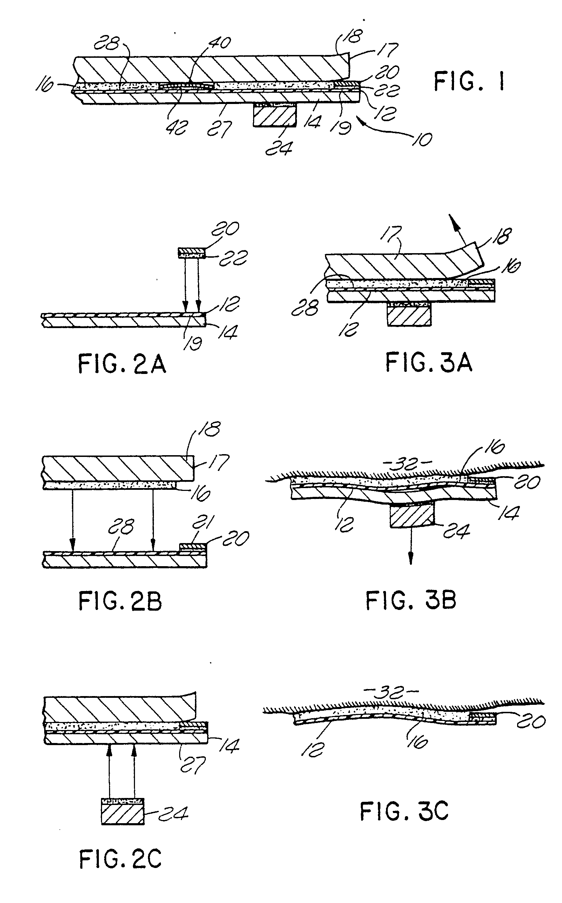

The present invention comprises a delivery system for enabling a film sheet to be adhesively affixed to a selected part of the body of a biological subject. The invention is particularly useful in allowing a thin film sheet which is stretchable and which has an adhesive applied to one side to be applied without clinging or sticking to itself prior to affixation. Accordingly, referring to FIGURE 1, a delivery system 10 for self-adhering copolymer medical covering comprises a film sheet 12 disposed on a film sheet carrier 14 by any suitable means such as casting or extrusion whereby the film sheet 12 adheres to the carrier 14 without the necessity of adhesives.

In the preferred embodiment, the film sheet is a thermoplastic polyurethane having a thickness of about 0,0127 mm or larger. Films having thicknesses of about 0,0127 mm are preferably made by casting a solution of the polyurethane or other copolymer onto a smooth surface such as a release coated silicon paper. Suitable materials and their production are described in US—A—2,871,218. The present invention is particularly useful with copolymer film sheets because of the tendency of such sheets to gather and cling to themselves making it impossible to affix such film sheets over a selected part of a biological subject. Such gathering and self-sticking thus prevents a continuous contact over the selected part of the biological subject allowing gaps and spaces, in which germs, dirt and other foreign matter can become trapped. Such unsterile conditions can defeat the objectives of the adhesive material of US—A—3 645 835 in actual use.

The transfer adhesive 16 is deposited over the subject facing surface 28 of the film sheet 12 by pressing (laminating) the transfer adhesive 16 on release sheet 17 against film sheet 12. The delivery system in accordance with the invention further includes a transfer adhesive initially disposed on a release sheet 17. Thereafter the release sheet 17 remains in place to cover and protect the transfer adhesive 16 until a time just prior to application to the selected part of the biological subject.

The transfer adhesive 16 may be disposed over the entire surface 28 of the film sheet 12 with the release backing sheet 17 similarly protecting the entire surface of the transfer adhesive 16. However, in the preferred embodiment, the release sheet 17 extends beyond an edge of the transfer adhesive 16 to form a flap section 18. The flap section 18 enables the release sheet 17 to be readily removed from the transfer adhesive 16 to expose the transfer adhesive 16 immediately prior to application to the selected part of the biological subject.

In the preferred embodiment, the transfer adhesive 16 is applied to cover only a portion of the subject facing surface 28 of the film sheet 12 with at least an edge region 19 being left without adhesive covering. This adhesive-free strip or edge region 19 then provides a region that can be grasped and pulled to facilitate easy removal of the film sheet 12 from the subject after use. Of course, other areas of the subject facing surface may be left without adhesive covering so that a gauze 40 or other object can be adhesively attached to the film by an adhesive 42 without itself being subsequently covered with transfer adhesive 16.

In accordance with the invention, the transfer adhesive 16 may be any of a number of well-known transfer adhesives. The release sheet 17 may be release-coated silicon or any other suitable release sheet well known in the art. The film sheet carrier may similarly be a release-coated silicon paper or preferably is a "Mylar" (registered trademark) sheet which is flexible but relatively rigid compared with the film sheet 12 and is relatively unstretchable when compared with the stretchability of the film sheet 12. Thus, the film sheet carrier 14 serves as a support for the film sheet 12 until after affixation of the film sheet 12 to the selected part of the biological subject. After the film sheet 12 has been affixed to the biological subject, the film sheet carrier 14 can be removed since its support function is no longer required.

It will be appreciated that the edge region 19 of the film sheet 12 may be provided as a pulling section without any reinforcement. However, in the preferred embodiment and in particular when the film sheet 12 is thin, a reinforcing pull strip 20 is laminated along the edge region 19 to thereby reinforce the edge region 19 and further facilitate removal of the film sheet 12 from the subject after use. The pull strip 20 may be adhesively affixed along the edge region 19 of the film sheet 12 utilizing any of a number of available permanent medical grade adhesives 22 such as medical grade transfer adhesive No. 1514 produced by 3M Corporation. The flap section 18 of the release backing sheet 16 then extends either partially or entirely over the pull strip 20. To facilitate removal of the film sheet carrier 14 from the film sheet after affixation, a carrier removal tab 24 is provided. The tab 24 is adhesively affixed to the back of the film sheet carrier 27 utilizing a suitable permanent adhesive which may, for example, be the same as the adhesive 22 used to affix the pull strip 20 to the subject facing surface 28 of the film sheet 12. In the preferred embodiment, the carrier removal tab 24 has a thickness of between 5 and 10 times the thickness of the film sheet carrier 14 and may, for example, be a tag with about 0,127 mm thickness. Such a thickness results in a carrier removal tab 24 which is relatively rigid compared to the film sheet carrier 14, thus enabling the carrier removal tab 24 to be grasped and pulled to separate and remove the film sheet carrier 14 from the film sheet 12 after the film sheet 12 has been applied to the selected part of the biological subject.

Referring to FIGURES 2A, 2B, and 2C, the delivery system for self-adhering copolymer medical coverings may be manufactured by first casting the polyurethane film 12 on a "Mylar" (registered trademark) backing 14. The combination of the polyurethane film 12 and Mylar backing 14 is commercially available and may be purchased from any of a number of different suppliers. A pull strip 20 with the permanent adhesive 22 on one side is then disposed on the edge region 19 of the film sheet 12 utilizing suitable laminating machinery such as the "Mark Andy 2100 System" (registered trademark) produced by Mark Andy Incorporated of Chesterfield, Missouri.

After the pull strip 20 has been permanently adhesively affixed to the edge of the film sheet 12, a release sheet 17 with transfer adhesive 16 disposed over all but a flap section 18 is laminated onto the subject facing surface 28 of the film sheet 12 as shown in FIGURE 2B so that the layer of transfer adhesive 16 is laterally adjacent to the previously affixed pull strip 20. Thus, at least a portion and preferably all of the top 21 of the pull strip 20 will be free of the transfer adhesive 16. In such an arrangement, the transfer adhesive 16 is laterally adjacent to the pull strip 20 with the release sheet 17 covering the transfer adhesive 16 and remaining in place until just prior to affixation to the selected part of the biological subject. The transfer adhesive and release backing may be disposed on the subject facing surface 28 of the film sheet 12 utilizing conventional laminating techniques and machinery and may be affixed in a manner similar to the method by which the pull strip 20 is affixed. In a similar manner a gauze 40 or other object can also be affixed to the film sheet without being covered by transfer adhesive.

Finally, referring to FIGURE 2C, the carrier removal tab 24 is permanently adhesively affixed to the back surface 27 of the film sheet carrier 14 utilizing a laminating technique as above described. It will, of course, be appreicated that the carrier removal tab 24 may be positioned at any location along the back surface 27 of the film sheet carrier 14. The resultant preferred embodiment of the delivery system 10 for self-adhering copolymer medical coverings with the pull strip 20 and the carrier removal tab 24 is thus shown in FIGURE 1.

Referring now to FIGURES 3A, 3B, and 3C, the use of the delivery system in accordance with the invention is illustrated. Specifically, the release sheet 17 is first removed by pulling on the flap section 18. After the release sheet 17 is removed and discarded, the transfer adhesive 16 which remains on the subject facing surface 28 of the film sheet 12 is exposed. The exposed transfer adhesive 16 is then placed over a selected part of a biological subject 32 as shown in FIGURE 3B. The polyurethane is thereby adhesively affixed to the biological subject 32 using the transfer adhesive 16 with the film sheet carrier 14 still in place. Once the film sheet 12 has been so adhesively affixed, the carrier removal tab 24 is pulled causing the film sheet carrier 14 to separate from the film sheet 12. The film sheet carrier 14 is then discarded. The part of the body of the biological subject 32 will thus be covered by the polyurethane film sheet 12 which is affixed by the adhesive 16 in a smooth continuous manner without the normal problems of gathering and self-sticking of the film sheet 12 prior to affixation.

The film sheet 12 may subsequently be removed by simply grasping the pull strip 20 and pulling the polyurethane film sheet 12 from the subject 32 as illustrated in FIGURE 3C.

## Claims

1. A delivery system (10) for self-adhering copolymer medical coverings for covering parts of biological subjects comprising:

a removable film sheet carrier (14);

a film sheet (12) for covering a selected part of the biological subject, the film sheet (12) having a subject facing surface (28) and a back surface, the back surface in adherence to the film sheet carrier, the film sheet carrier (14) for supporting the back surface of the film sheet (12) until after the film sheet (12) is applied to cover the selected part of the biological subject;

a release sheet (17); and

a transfer adhesive (16) disposed for covering at least a portion of the release sheet (17), the release sheet (17) and the transfer adhesive (16) affixed to the subject facing surface (28) of the film sheet (12) whereby the transfer adhesive (16) adheres to at least a portion of the subject facing surface (28) so that the film sheet (12) is sandwiched between the film sheet carrier (14) on the back surface and the transfer adhesive (16) on the subject facing surface (28) prior to application to the biological subject, whereby the transfer adhesive (16) will be on the subject facing surface (28) of the film sheet (12) upon removal of the release sheet (17) in preparation for affixation of the film sheet (12) to the selected part of the biological subject, the film sheet (12) being thereafter adhesively held to the selected part of the biological subject by the transfer adhesive (16), wherein possibly a gauze (40) is affixed to a section of the film sheet (12) and an adhesive (42) for affixing the gauze (40) to the section of the film sheet (12),

characterized by a non-self-supporting thinness of the film sheet (12) disposed on the film sheet carrier (14), whereby the film sheet (12) releasibly, non-adhesively adheres to the film sheet carrier (14) over the entire back surface (28) of the film sheet (12), the film sheet carrier (14) being flexible with and inelastic relative to the film sheet (12), to support the film sheet 12 but conform generally to the contours of the part of the biological subject during application of the film sheet (12) to the biological subject, the film sheet (12) and film sheet carrier (14) being of the same size, whereby the film sheet (12) is coextensive with the film sheet carrier (14) whereby the film sheet carrier (14) supports the entire back surface of the film sheet (12) until after the film sheet (12) is affixed to cover the selected part of the biological subject, the film sheet carrier (14) thereafter being removed.

2. The delivery system (10) of claim 1 further comprising:

a pull strip (20) adhesively attached along at least a section of one edge of the subject facing surface (28) of the film sheet (12).

3. The delivery system (10) of claim 1, further comprising an adhesive free section of the release sheet (17) thay may be provided at a position which lies over the gauze (40), which may be provided on the film sheet (12).

4. The delivery system (10) of Claim 1 further comprising:

a carrier removal tab (24) attached to the film sheet carrier (14) for being grasped and pulled to thereby pull the film sheet carrier (14) from the back surface of the film sheet (12) after placement of the film sheet (12) on the selected part of the biological subject, the carrier removal tab (24) being positioned so that the film sheet carrier (14) is between the film sheet (12) and the carrier removal tab (24).

5. The delivery system (10) of Claim 4 wherein the carrier removal tab (24) is permanently adhesively attached to the film sheet carrier (14).

## Patentansprüche

1. Handhabungseinheit (10) für selbsthaftende, copolymere medizinische Abdeckungen zum Abdecken von Teilen von biologischen Objekten, mit:

einem entfernbaren Filmschichtträger,

einer Filmschicht (12) zum Abdecken von ausgewählten Teilen eines biologischen Objektes, wobei die Filmschicht (12) eine dem Objekt zugewandte Seite (28) und eine Rückseite aufweist, die an dem Filmschichtträger (14) haftet, welcher die Rückseite der Filmschicht (12) versteift, bis die Filmschicht zum Abdecken der ausgewählten Teile des biologischen Objektes verwendet wird, einer Schutzfolie (17) und

einer Klebeschicht (16), die wenigstens einen Teil der Schutzfolie (17) bedeckt, wobei Schutzfolie (17) und Klebeschicht (16) auf der dem Objekt zugewandten Seite (28) der Filmschicht (12) befestigt sind und die Klebeschicht (16) an wenigstens einem Teil der dem Objekt zugewandten Seite (28) haftet, so daß die Filmschicht (12) zwischen dem Filmschichtträger (14) auf der Rückseite und der Klebeschicht (16) auf der dem Objekt zugewandten Seite (28) vor der Benutzung am biologischen Objekt eingeschlossen ist und wobei sich nach dem Abziehen der Schutzfolie (17) zur Vorbereitung der Befestigung der Filmschicht (12) auf den ausgewählten Teilen des biologischen Objekts die Klebeschicht (16) auf der dem Objekt zugewandten Seite (28) der Filmschicht (12) befindet und danach die Filmschicht (12) mittels der Klebeschicht (16), innerhalb der eine Gaze (40) an einem Bereich der Filmschicht (12) mittels eines Klebers (42) befestigt sein kann, an den ausgewählten Bereiches des biologischen Objektes gehalten wird,

gekennzeichnet durch eine sehr geringe, nicht mehr selbst versteifende Stärke der auf dem Filmschichtträger (14) angebrachten Filmschicht (12), wobei die Filmschicht (12) lösbar, nicht klebend mit ihrer gesamten Rückseite am Filmschichtträger (14) haftet und der Filmschichtträger (14) zwar mit der Filmschicht (12) flexibel, aber relativ zu dieser unelastisch ist, wodurch die Filmschicht (12) versteift wird, sich jedoch während der Anbringung am biologischen Objekt an die Oberflächenform des Teiles des biologischen Objekts anpaßt und wobei Filmschicht (12) und Filmschichtträger (14) gleich groß sind, so daß Filmschicht (12) und Filmschichtträger (14) eine gemeinsame Erstreckung aufweisen, wodurch der Filmschichtträger (14) die gesamte Rückseite der Filmschicht (12) versteift, bis danach die Filmschicht (12) zum Abdecken der ausgewählten Teile des biologischen Objektes befestigt und der Filmschichtträger (14) entfernt wird.

2. Handhabungseinheit (10) nach Anspruch 1, gekennzeichnet durch einen Abziehstreifen (20), der entlang wenigstens einer Kante der dem Objekt zugewandten Seite (28) der Filmschicht (12) aufgeklebt ist.

3. Handhabungseinheit (10) nach Anspruch 1, gekennzeichnet durch einen klebefreien Bereich der Schutzfolie (17), der in dem Bereich vorgesehen werden kann, der über der Gaze (40) liegt, die auf der Filmschicht (12) vorgesehen werden kann.

4. Handhabungseinheit (10) nach Anspruch 1, gekennzeichnet durch eine Entfernungslasche (24) zum Festhalten und Ziehen, die am Filmschichtträger (14) befestigt ist, um damit den Filmschichtträger (14) von der Rückseite der Filmschicht (12) abzuziehen, nachdem die Filmschicht (12) auf den ausgewählten Teilen des biologischen Objektes plaziert wurde, wobei die Entfernungslasche (24) so angeordnet ist, daß der Filmschichtträger (14) zwischen der Filmschicht (12) und der Entfernungslasche (24) liegt.

5. Handhabungseinheit (10) nach Anspruch 4, dadurch gekennzeichnet, daß die Entfernungslasche (24) dauerhaft am Filmschichtträger (14) klebt.

**Revendications**

1. Système de transfert (10) pour pansements copolymères autocollants pour recouvrir certaines parties de sujets biologiques, comprenant:
   un support amovible (14) de feuille mince;
   une feuille mince (12) destinée à recouvrir une partie choisie du sujet biologique, la feuille mince (12) présentant une surface (28) faisant face au sujet et une surface arrière, la surface arrière adhérant au support de feuille mince, le support (14) de feuille mince étant destiné à supporter la surface arrière de la feuille mince (12) jusqu'à ce que la feuille mince (12) soit appliquée pour recouvrir la partie choisie du sujet biologique;
   une feuille anti-adhérente (17); et
   un adhésif (16) de transfert disposé de façon à recouvrir au moins une partie de la feuille anti-adhérente (17), la feuille anti-adhérente (17) et l'adhésif de transfert (16) étant fixées à la surface (28), faisant face au sujet, de la feuille mince (12) de manière que l'adhésif (16) de transfert adhère à au moins une partie de la surface (28) faisant face au sujet pour que la feuille mince (12) soit prise en sandwich entre le support (14) de feuille mince sur la surface arrière et l'adhésif de transfert (16) sur la surface (28) faisant face au sujet avant l'application au sujet biologique, de façon que l'adhésif (16) de transfert se trouve sur la surface (28) faisant face au sujet de la feuille mince (12) après l'enlèvement de la feuille anti-adhérente (17) en préparation à la fixation de la feuille mince (12) sur la partie choisie du sujet biologique, l'adhésif (16) de transfert maintenant ensuite par adhérence la feuille mince (12) sur la partie choisie du sujet biologique, une gaze (40) pouvant être fixée à une zone de la feuille mince (12) et un adhésif (42) pouvant fixer la gaze (40) à la zone de la feuille mince (12),
   caractérisé par le fait que la feuille mince (12) disposée sur le support (14) de feuille mince est d'une minceur la rendant non auto-portante, de manière que la feuille mince (12) adhère de façon amovible, non adhésive, au support (14) de feuille mince sur toute la surface arrière (28) de la feuille mince (12), le support (14) de feuille mince étant flexible avec la feuille mince (12) et étant inélastique par rapport à elle, afin de supporter la feuille mince (12), mais d'épouser globalement le contour de la partie du sujet biologique pendant l'application de la feuille mince (12) sur le sujet biologique, la feuille mince (12) et le support (14) de feuille mince étant des mêmes dimensions, en sorte que la feuille mince (12) et le support (14) de feuille mince sont superposables de façon que le support (14) de feuille mince supporte toute la surface arrière de la feuille mince (12) jusqu'à ce que la feuille mince (12) soit fixée pour recouvrir la partie choisie du sujet biologique, le support (14) de feuille mince étant ensuite enlevé.

2. Système de transfert (10) selon la revendication 1, comprenant en outre:

une bande (20) d'arrachement reliée de façon adhésive le long d'au moins une zone d'un bord de la surface (28), faisant face au sujet, de la feuille mince (12).

3. Système de transfert (10) selon la revendication 1, comprenant en outre une zone sans adhésif de la feuille anti-adhérente (17) qui peut être prévu dans une position s'étendant au-dessus de la gaze (40) pouvant être prévue sur la feuille mince (12).

4. Système de transfert (10) selon la revendication 1, comprenant en outre:

une languette (24) d'enlèvement de support attachée au support (14) de feuille mince de façon à être saisie et tirée pour tirer ainsi le support (14) de feuille mince de la surface arrière de la feuille mince (12) après mise en place de la feuille mince (12) sur la partie choisie du sujet biologique, la languette (24) d'enlèvement du support étant positionnée de façon que le support (14) de feuille mince se trouve entre la feuille mince (12) et la languette (24) d'enlèvement du support.

5. Système de transfert (10) selon la revendication 4, dans lequel la languette (24) d'enlèvement du support est attachée de façon permanente, par adhésif, au support (14) de feuille mince.

FIG. 1

FIG. 2A

FIG. 3A

FIG. 2B

FIG. 3B

FIG. 2C

FIG. 3C